# EUROPEAN PATENT SPECIFICATION

(11) **EP 3 415 119 B1**
(45) Date of publication and mention of the grant of the patent: **06.05.2026**
(21) Application number: 18168180.0
(22) Date of filing: 14.11.2011
(51) Int. Cl.: A61F 2/24

(54) **PROSTHESIS FOR CARDIOVASCULAR VALVE**
PROTHESE FÜR KARDIOVASKULÄRE KLAPPE
PROTHÈSE POUR VALVE CARDIOVASCULAIRE

(30) Priority: 12.11.2010 IT MI20102102
(43) Date of publication of application: 19.12.2018
(62) Divisional of application: 11805596.1
(73) Proprietor: Innovheart S.r.l., 20123 Milano (MI) (IT)
(72) Inventor: RIGHINI, Giovanni, 1196 Gland (CH); ZANON, Sarah, 1196 Gland (CH)
(74) Representative: Provvisionato, Paolo

(56) References cited:
- WO-A2-2008/029296
- FR-A1- 2 874 813
- US-A1- 2008 077 235

## Description

The solution in accordance with one or more embodiments of the present invention relates to the field of prostheses. More specifically, that solution relates to prostheses for cardiovascular valves.

Prostheses for cardiovascular valves are used to fulfil the normal physiological function of making the flow of blood in the heart unidirectional.

Implanting a prosthesis for a cardiac valve is a fairly complex intervention, generally carried out in open heart conditions. Furthermore, to that end, the natural beating of the heart is often interrupted, using extra-corporeal circulation of the blood or using artificial systems for pumping the blood. Percutaneous transcatheter techniques have also been used recently, wherein the prosthesis is implanted in the heart with transluminal access via a peripheral inlet with low invasiveness, for example, starting from the femoral arteries. To that end, the prosthesis is radially compressed; a guidance system, generally a catheter, guides the prosthesis as far as the implantation site thereof; once the prosthesis has been correctly positioned, it is released by the guidance system and expands, for example, owing to resilient return or by a balloon positioned therein being inflated, so as to recover the operating dimensions thereof.

A problem of known prostheses for cardiac valves is anchoring them at the implantation site. In the case of implantation with a surgical technique, the prosthesis can be anchored by means of sutures in the tissue of the implantation site. It is also possible to use staples which are mounted on a main body thereof and which are secured to the tissue of the implantation site [cf. US 2008/0077235]. In the case of an implantation procedure with a transcatheter technique, the prosthesis can be anchored to the implantation site with pressure by means of a radial force which is directed outwards and which is applied to the annulus of the native valve by the prosthesis when it is expanded. Furthermore, the prosthesis can also be provided with an outer radial groove, in which the annulus is fixed, or teeth or hooks which become engaged in the tissue of the implantation site.

WO 2008/029296 discloses a prosthesis for a heart valve including a separate or combined anchor that folds around the malfunctioning native valve leaflets, sandwiching them in a manner so as to securely anchor the replacement valve in a precise, desired location.

FR 2874813 discloses a prosthesis for a heart aortic valve, made of two pieces with an anchor that is chirurgcally fastened to the heart walls.

In any case, with the prostheses of known type, it is difficult to obtain reliable anchoring which can prevent displacements and migrations of the prosthesis itself. Furthermore, it is difficult to ensure good sealing at the interface between the prosthesis and the implantation site which can prevent paravalvular losses with reflux of blood. It is also difficult to limit the impact of the prosthesis on the anatomy and/or physiology of the implantation site which could bring about negative interactions with the function of the prosthesis.

Those problems are exacerbated in the case of prostheses for the mitral valve. The mitral valve is subjected to greater differences of pressure than the other heart valves; this involves greater demands for reliability of the anchoring thereof at the implantation site. Furthermore, the mitral valve is positioned between two open spaces (that is to say, the left atrium and the left ventricle) so that it does not provide any interface with well-identified anatomical characteristics for anchoring the prosthesis. The anchoring of the prosthesis is also impeded by the presence of a very complex subvalvular apparatus (that is to say, chordae tendinae and papillary muscles). The most common pathology of the mitral valve is of a degenerative type, generally associated with prolapse of the annulus of the valve; therefore, the annulus of the damaged mitral valve does not provide a consistent and solid structure against which the prosthesis can expand and be anchored, but instead any radial force applied by the prosthesis to the annulus is counter-productive for the pathology of the mitral valve, making the anchoring of the prosthesis unstable and very unreliable over time. In addition, the implantation of a prosthesis without carrying out beforehand the removal of the damaged mitral valve may involve deformation of the mitral valve itself, with the risk of creating interference with the flow of arterial blood in systole, bringing about an effect known as Systolic Anterior Motion or SAM.

In general terms, the solution in accordance with one or more embodiments of the present invention is based on the idea of providing a prosthetic system with a system for anchorage on the valve leaflets of the cardiovascular valve whose functionality it is desirable to restore without it therefore being necessary to carry out precautionary removal thereof via the surgical route. This is carried out by means of separate structures which match each other in situ in order to anchor the prosthetic system to the valve leaflets of the cardiovascular valve.

In particular, one or more aspects of the solution in accordance with specific embodiments of the invention or the methods of use thereof are indicated in the independent claims with advantageous features of the same solution which are indicated in the dependent claims. More specifically, an aspect of the solution in accordance with an embodiment of the invention provides for a prosthesis for a cardiovascular valve, preferably an atrio-ventricular heart valve, such as the mitral valve. The prosthesis described herein comprises a valve structure for implementing a functionality of the cardiovascular valve (for example, a group of prosthetic leaflets). A support structure is provided in order to support the valve structure; the support structure has a peripheral wall which defines a through-hole for allowing a flow of blood through the support structure (for example, a ring to which the valve leaflets of the prosthesis are fixed). An anchoring member is used to anchor the support structure to an implantation site in the body of a patient, bringing about the integration of the prosthesis with the cardiovascular valve.

The anchoring member is separate from the support structure and comprises, for example, a complete or partial ring. The anchoring member co-operates with the peripheral wall of the support structure, extending round it so as to lock the valve leaflets of the cardiovascular valve between the anchoring member and the peripheral wall of the support structure.

Another aspect which is described provides for a delivery system for that prosthesis in order to guide it to the implantation site via the transluminal route and to anchor it on the valve leaflets of the cardiovascular valve.

Another aspect which is described relates to a procedure for restoring the functionality of a cardiovascular valve, comprising the steps of introducing, positioning and implanting such a prosthesis in the body of a patient.

Another aspect which is described relates to the use of such a prosthesis for restoring the functionality of a cardiovascular valve by the prosthesis being introduced, positioned and implanted in the body of a patient in such a manner as to entrap and lock the valve leaflets of the cardiovascular valve of the patient between the peripheral wall of the support structure and the anchoring member so as to keep the prosthesis in position.

Another aspect of the invention relates to a kit for restoring the functionality of a cardiovascular valve comprising such a prosthesis and instructions for introducing, positioning and implanting the prosthesis in the body of a patient in such a manner as to entrap and lock the valve leaflets of the cardiovascular valve of the patient between the peripheral wall of the support structure and the anchoring member so as to keep the prosthesis in position.

The solution in accordance with one or more embodiments of the invention will, as will other features and relevant advantages, be better understood with reference to the following detailed description which is given purely by way of non-limiting example and which is intended to be read together with the appended Figures (in which, for simplicity, corresponding elements are indicated with identical or similar reference numerals and the explanation thereof is not repeated). In that regard, it will be expressly understood that the Figures are not necessarily to scale (with some details which may be exaggerated and/or simplified) and that, unless otherwise indicated, they are simply used to conceptually illustrate the structures and procedures described. In particular:
Figure 1 is a partially sectioned view of a human heart in which the solution in accordance with an embodiment of the invention can be used,
Figure 2 is a schematic illustration with separated members of the prosthesis in accordance with an embodiment of the invention,
Figure 3 is a schematic cross-section of an example of use of the prosthetic system in accordance with an embodiment of the invention,
Figure 4A - Figure 4C show variants of the support structure of Figure 2,
Figure 5A - Figure 5I show variants of the anchoring member of Figure 2,
Figure 6A is a partial view of an embodiment of a delivery system for the anchoring member,
Figure 6B is a cross-section of a detail of the delivery system of Figure 6A,
Figure 7A - Figure 7C show the main steps of a procedure for the intra-ventricular implantation of the prosthesis of the invention,
Figure 8 shows an example of sub-annular implantation of the prosthesis of the invention,
Figure 9A - Figure 9B show an example of a procedure for the supra-annular implantation of the prosthesis of the invention and
Figure 10A - Figure 10C show another example of a procedure for supra-annular implantation of the prosthesis of the invention.

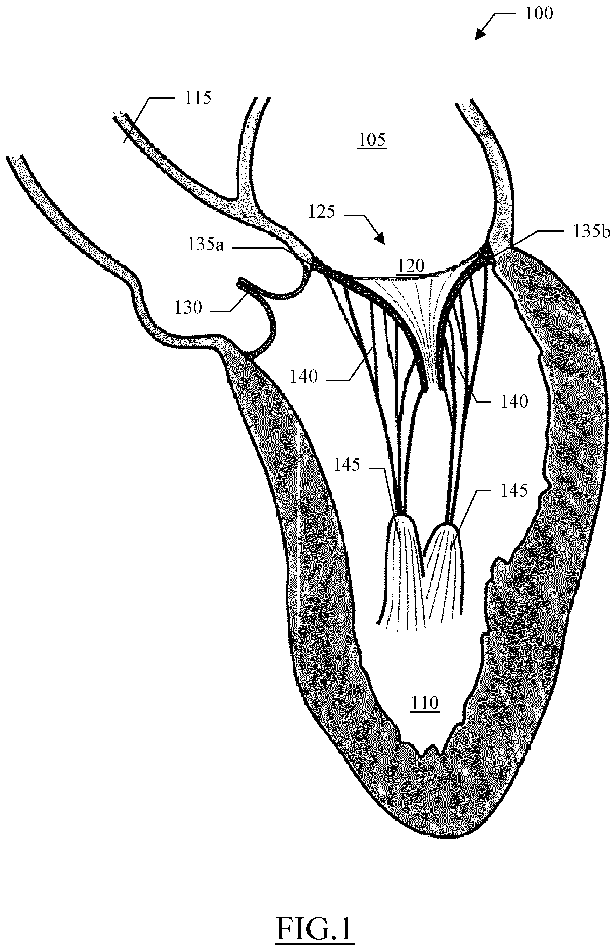

Figure 1 is a cross-section of the left side of a heart 100 which pumps arterial blood towards peripheral regions. The right side thereof is not shown in Figure 1 for simplicity. The left side of the heart 100 comprises the left atrium 105 which communicates with the left ventricle 110 via a left atrio-ventricular orifice 120. A left atrio-ventricular valve 125, the mitral or bicuspid valve, is arranged in the region of the left atrio-ventricular orifice 120 in order to make the flow of arterial blood unidirectional from the left atrium 105 to the left ventricle 110; an aortic valve 130 is arranged between the left ventricle 110 and the aorta 115 in order to make the flow of arterial blood unidirectional from the left ventricle 110 to the aorta 115.

The mitral valve 125 comprises two valve leaflets 135a and 135b which are connected to internal walls of the heart 100 in the region of the left atrio-ventricular orifice 120 by means of an annulus which comprises a fibrous ring which defines a geometric contour of the mitral valve 125. The valve leaflets 135a, 135b are the movable elements, that is to say, flexible elements, of the mitral valve 125 which are used to intercept the arterial blood.

The mitral valve 125 also has a sub-valvular apparatus which is composed of chordae tendinae 140 which connect the free edge of both the valve leaflets 135a and 135b to the papillary muscles 145 which are connected to the internal wall of the left ventricle 110. The chordae tendinae 140 and the papillary muscles 145 contribute to opening and closing the mitral valve 125 and further prevent the valve leaflets 135a, 135b from prolapsing in the left atrium 105.

The tricuspid valve (in the right side of the heart 100 not shown in the Figure) has a similar anatomical structure with valve leaflets (three in this case) which are connected to the internal walls of the heart by means of an annulus and with a sub-valvular apparatus which is composed of chordae tendinae and papillary muscles.

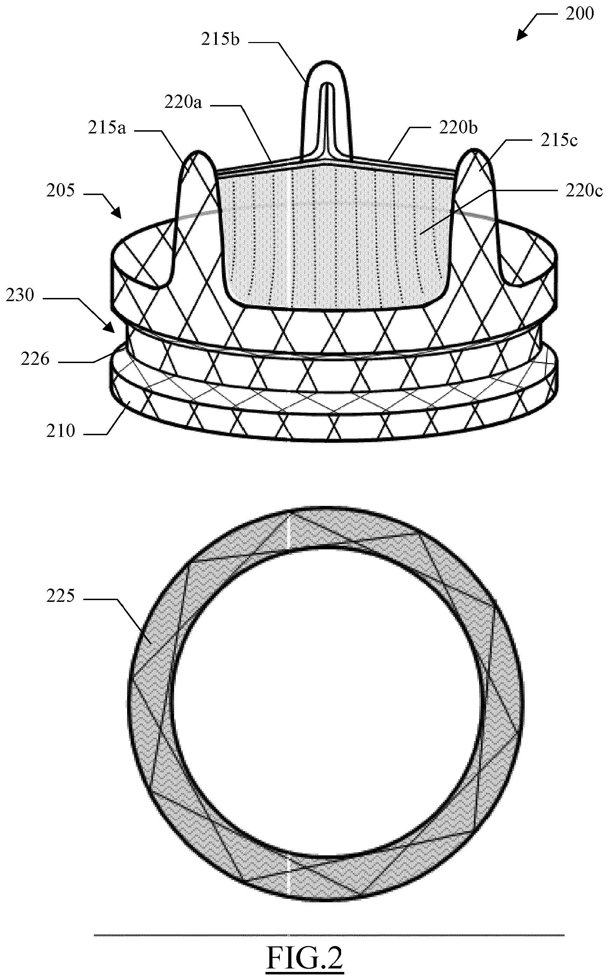

A prosthesis 200 is shown in Figure 2 and comprises a prosthesis member 205 which comprises a support structure 210 with a through-hole for allowing the passage of blood therein. For example, in the embodiment illustrated in the Figure, the support structure 210 is of annular shape and is preferably provided with attachment brackets 215a, 215b, 215c which extend axially from an edge thereof and which are preferably arranged in an equidistant manner along it.

Prosthetic leaflets 220a, 220b, 220c are each fixed to the framework 210 between two brackets 215a - 215c, respectively. The prosthetic leaflets 220a - 220c are constructed from flexible material which may be synthetic (for example, silicone-containing elastomer material, polyurethane-containing elastomer material, or combinations thereof) or biological (for example, pericardium of animal origin). The prosthetic leaflets 220a - 220c can move apart freely in order to open the hole of the framework 205 and may become partially superimposed in order to close the through-hole of the framework 205.

The prosthesis 200 also comprises an anchoring member 225 which is separate from the prosthetic member 205 and which can be connected to the framework 210 in a stable manner.

In the embodiment of Figure 2, the anchoring member 225 is a substantially circular ring. The framework 210 has a seating for the ring 225 when it is fitted around the framework 210; for example, in the form of a circumferential groove 226 on an outer surface of the peripheral wall of the framework 210 so as to define a channel 230 conjugate to the ring 225. The framework 210 and the ring 225 are configured and have dimensions so that they are connected with interference so as to lock the ring 225 in the channel 230, in particular with valve leaflets being interposed between them and thereby being locked as described in greater detail below.

The anchoring member 225 is used to anchor, when it matches the framework 210, the prosthesis 200 to the leaflets of the atrio-ventricular valve. For the simplicity of the specification, reference will always be made below to the application of the prosthesis system 200 to the mitral valve; similar considerations also apply to the application of the prosthesis system to the tricuspid valve in the right side of the heart.

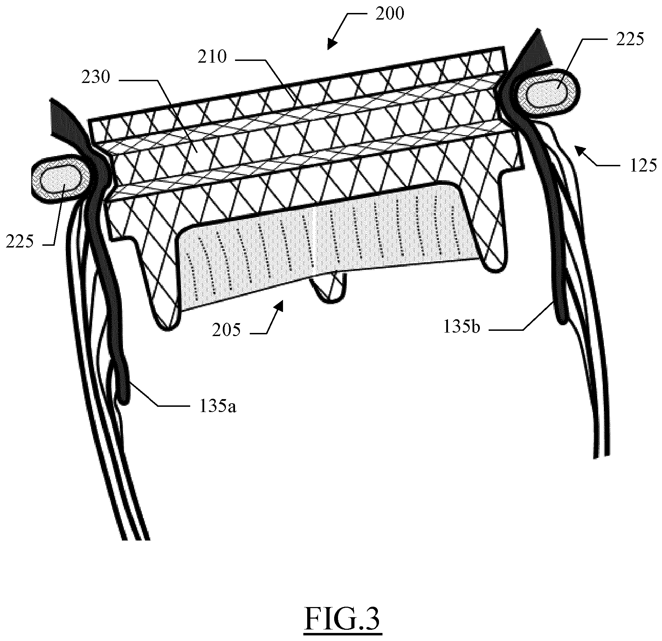

Generally, the prosthetic member 205 and the ring 225 are arranged at opposite sides of the valve leaflets of the mitral valve 125. When the ring 225 is matched to the framework 210, the valve leaflets of the mitral valve 125 are locked between the framework 210 and the ring 225. For example, in the embodiment illustrated in Figure 3, the prosthetic member 205 is arranged inside the valve leaflets 135a, 135b and the ring 225 is arranged at the outer side thereof so as to extend completely round them. The ring 225 is fitted on the framework 210 so as to be partially introduced into the channel 230; in that manner, the valve leaflets 135a, 135b remain locked between the fitting 210 and the ring 225.

The anchoring mechanism described above in accordance with an embodiment of the invention makes the prosthesis system 200 integral with the mitral valve 125, structurally embedding a significant portion thereof. This allows to provide a reliable anchoring of the prosthesis 200 on the mitral valve 125 so as to prevent any risk of dislodgement and migrations of the prosthesis 200. Consequently, the mitral valve 125 itself becomes an essential component for the correct functioning of the prosthesis 200. The anchoring is independent of the anatomical characteristics of the annulus so that it is possible to have stable and reliable anchoring over time even in the presence of a prolapse thereof. Furthermore, the mechanical integration thereby obtained between the prosthesis 200 and the mitral valve 125 provides optimum sealing at an interface between the prosthesis 200 and the mitral valve which prevents or at least substantially reduces any risk of paravalvular reflux of arterial blood at that interface.

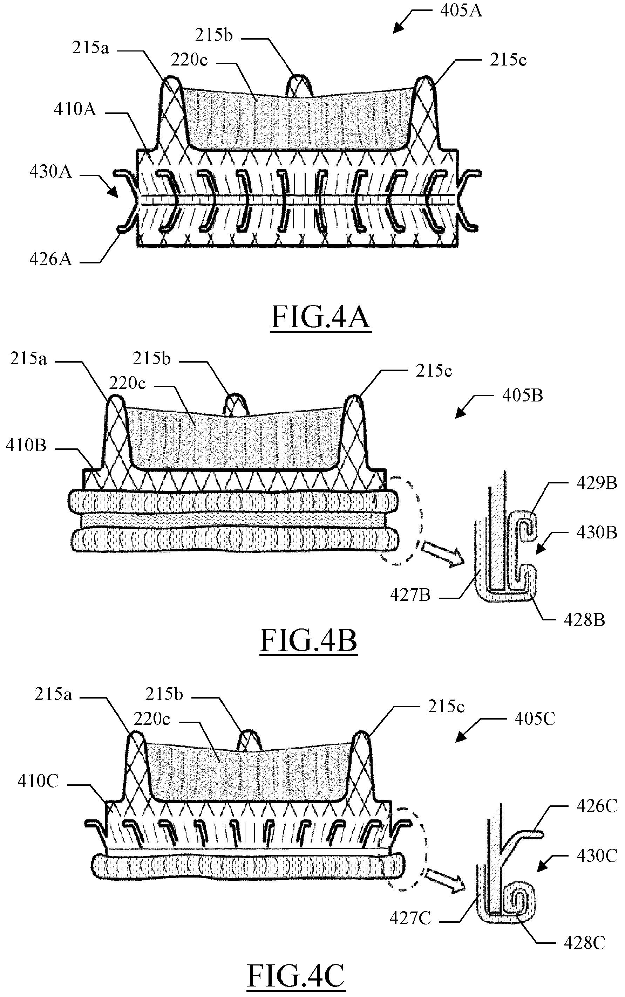

The above-described solution can be implemented with various structures both of the prosthetic member and of the anchoring member. In particular, embodiments of the prosthetic member in accordance with various embodiments of the invention are shown in Figure 4A - Figure 4C.

The prosthetic member 405A of Figure 4A further comprises a framework 410A which is of circular ring-like shape and whose outer lateral surface is provided with protrusions 426A (for example, from 10 to 30 protrusions) which project radially therefrom; each protrusion 426A has a C-like shape which is defined by two opposing fingers. The protrusions 426A are successively arranged along the peripheral wall of the framework 410A so as to define the outline of a channel 430A which brings about the seating of the ring 225 (not shown in the Figure). That structure allows the ring to be engaged on the framework 410A so as to improve the stability of their connection. Furthermore, in that case, it is also possible to carry out a procedure for connecting the framework 410A to the ring according to a defined sequence of steps. In particular, the protrusions 426A are initially deflected axially so as to move into abutment with the outer lateral surface of the framework 410A so as to facilitate the delivery of the prosthetic member 405A to the implantation site. At this point, it is possible to bring about selective release of groups of fingers at different times in order to engage the ring; for example, it is possible to release all the upper fingers of the protrusions 426A, to engage the ring 225 with those fingers and subsequently to release all the lower fingers for the final engagement of the ring.

With reference to Figure 4B, a prosthetic member 405B further comprises a circular ring-like framework 410B whose outer lateral surface is smooth. An annular face 427B covers a lower free edge of the framework 410B; the face 427B may be of synthetic material (for example, PET or PTFE) or of biological material (for example, pericardium of animal origin). The face 427B is folded about itself at the outer side of the framework 410B so as to form a lower annular lip 428B and an upper annular lip 429B which define a channel 430B for seating the ring 225 (not shown in the Figure). That structure reduces the tensions of the framework 410B which are caused by the ring 225 on the valve leaflets which are entrapped during use, further promoting the integration thereof via controlled endothelisation thereof.

In Figure 4C, the prosthetic member 405C further comprises a circular ring-like framework 410C whose outer lateral surface is provided with a group of fingers 426C (for example, from 10 to 30 fingers) which project radially therefrom; each finger 426C is orientated upwards and terminates in an end which is folded radially outwards. An annular face 427C of synthetic or biological material covers a lower free edge of the framework 410C and is folded about itself at the outer side of the framework 410C so as to form a lower annular lip 428C below the fingers 426C. In that manner, the fingers 426C and the lip 428C define a channel 430C which forms the seating of the ring 225 (not shown in the Figure). That structure combines the advantages provided by the fingers 426C of Figure 4A with the advantages provided by the face 426C of Figure 4B. The arrangement of the fingers 426C and the annular lip 428C may be transposed even if the solution illustrated above is the most suitable for withstanding the load generated by the pressure gradient applied to the prosthetic member 405C during the systolic phase.

The seating for the ring 225 preferably has a flared cross-section, for example, with an inclination of from 30° to 60° with respect to a radial direction. The flared form facilitates fitting of the ring when it is coupled to the framework; furthermore, the connection between the ring and the seating is self-aligning so as to automatically correct, within reasonable limits, any inaccuracies of mutual positioning of the ring and the seating during the procedure for anchoring the prosthesis.

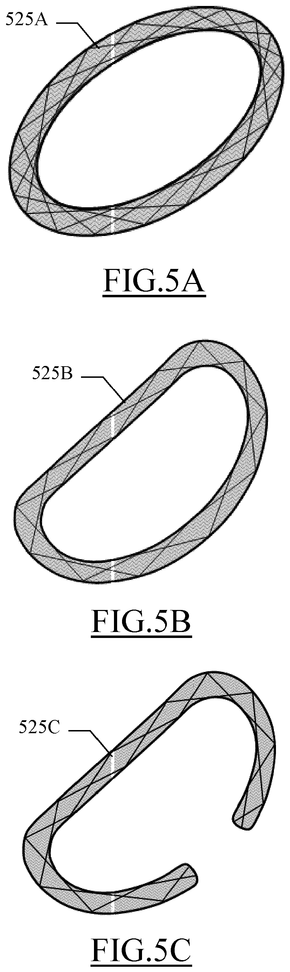

Variants of the anchoring member are shown in Figure 5A to Figure 5I.

In Figure 5A, the anchoring member 525A is of substantially oval shape and in Figure 5B the anchoring member 525B is of substantially D-like or "bean"-like shape, that is to say, substantially an oval shape flattened at one side. The embodiment of the circular anchoring member 225 of Figure 2 promotes the structural simplicity and simplicity of connection of the prosthesis, which is also circular, to the framework. The other embodiments of the anchoring member of Figure 5A and Figure 5B promote the correspondence to the anatomy of the atrio-ventricular valves, imitating the natural shape of the annulus of the tricuspid valve and the mitral valve, respectively.

The anchoring member 525C of Figure 5C is further substantially D-shaped, but has an opening at the centre of a curved portion thereof having an extent of preferably from 20 to 60% of the length of the rectilinear side.

The closed configuration of the anchoring members illustrated in Figure 2, Figure 5A, Figure 5B promotes the stability and the sealing of the connection with respect to the framework. However, the open structure of the anchoring member illustrated in Figure 5C promotes its simplicity of positioning around the atrio-ventricular valve, particularly in specific types of implant, as will be described in greater detail below.

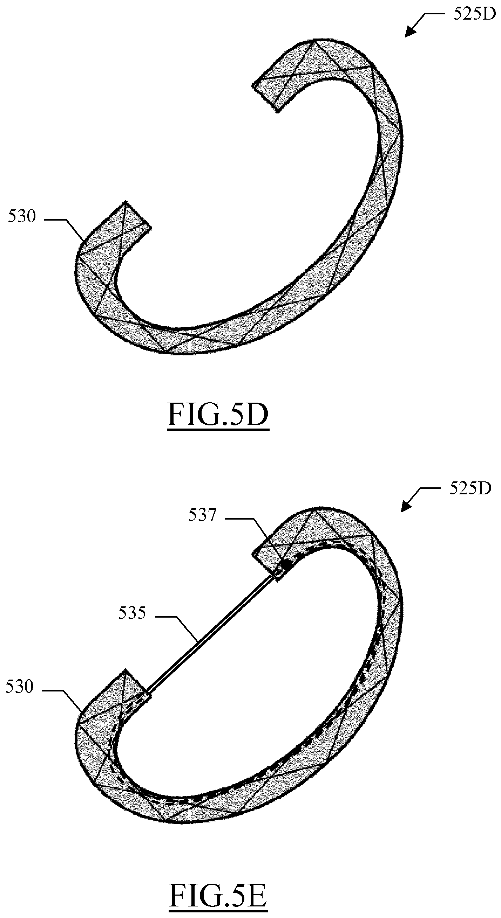

With reference to Figure 5D, the anchoring member 525D comprises a main structure 530 with an open configuration, for example, of D-like shape, with an opening at the centre of the substantially rectilinear side thereof, which extends, for example, over approximately from 40 to 70% of the length thereof. The main structure 530 is tubular with an internal cavity which extends over the entire length thereof.

The main structure 530 may be closed after being positioned around an atrio-ventricular valve by means of a closure wire 535 (see Figure 5E), for example, a guidewire of polymer or metallic material, of the type commonly used in cardiological intervention. To that end, using a technique of snaring which is known per se, the closure wire 535 which terminates in an engagement cap is threaded inside the cavity of the main structure 530 at a free end thereof, is caused to pass through the entire cavity, is captured by means of the engagement cap thereof at the other free end of the main structure 530, is reintroduced into the cavity at the preceding free end, being closed on itself, optionally also being superimposed several times by repeating the same operations as set out above, and is then locked by means of a locking structure 537, for example, a metallic clip or the like. Before the closure wire 535 is locked, it is also possible to apply a tension which clamps the closure wire 535 to the main structure 530 and which can also deform it, moving the free ends thereof towards each other, at a maximum until they are moved into contact. In that manner, the closure wire 535 restores structural continuity which confers a closed configuration on the anchoring member 525D again.

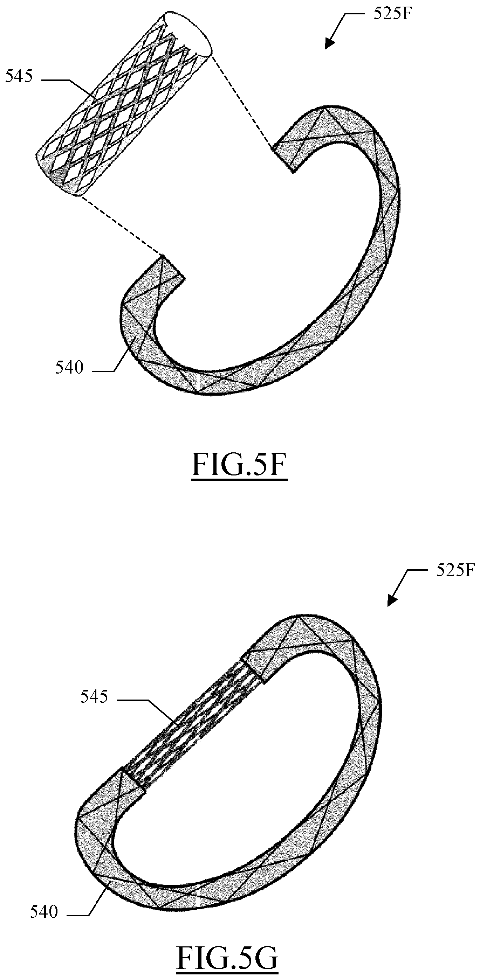

With reference to Figure 5F, the anchoring member 525F comprises a main structure 540 which is substantially similar to that of the anchoring member 525D of Figure 5D. The anchoring member 525F further comprises a closure structure 545 which is separate from the main structure 540 and which is composed of, for example, deformable material and has a shape corresponding to that of the cavities of the main structure 540 (that is to say, cylindrical in the embodiment in question), with a length and width at rest greater than the extents of the opening and of the tubular cavity of the main structure 540, respectively.

When the main structure 540 is positioned around the atrio-ventricular valve, the closure structure 545 is positioned so as to straddle the opening of the main structure 540 (Figure 5G), for example, using a guidewire to navigate inside the cavity of the main structure 540. Therefore, each end of the closure structure 545 is inserted inside a corresponding end of the tubular cavity of the main structure 540; the closure structure 545 expands, for example, by resilient return or by a balloon positioned therein being inflated, so as to become embedded in the main structure 540. In that manner, the closure structure 545 acts as a bridge between the free ends of the main structure 540, with it restoring structural continuity which confers a closed configuration on the anchoring member 525F again.

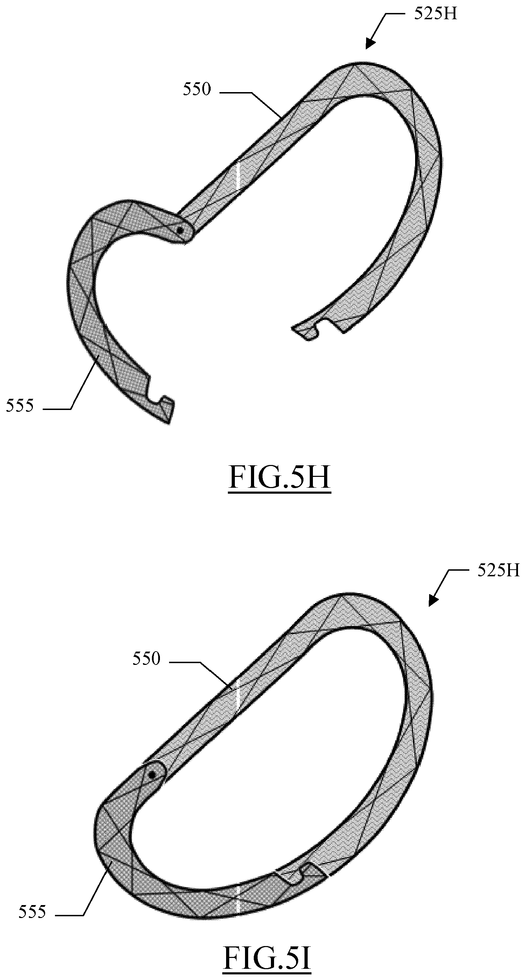

With reference instead to Figure 5H, the anchoring member 525H comprises a main structure 550 and a closure structure 555 which are articulated to each other. For example, in the embodiment illustrated in the Figure, the main structure 550 is a half-ring which reproduces a greater portion of a D-like shape, and the closure structure 555 is a half-ring which reproduces a lesser portion of the same D-like shape; the half-rings 550 and 555 terminate at the free ends thereof with complementary hooks. In the condition shown in the Figure, the free end of the half-ring 555 is moved away from the free end of the half-ring 550 so as to confer an open configuration on the anchoring member 525H.

Referring to Figure 5I, once the anchoring member 525H has been positioned around the atrio-ventricular valve, the half-ring 555 is rotated with respect to the half-ring 550 until it engages therewith. It is also possible to add a closure wire inside the anchoring member 525H when it is tubular in order to lock the half-rings 550 and 555 in the closed condition. The composite structure of the anchoring members of Figure 5D - Figure 5E, Figure 5F - Figure 5G and Figure 5H - Figure 5I allows provision of an open configuration during positioning thereof around the atrio-ventricular valve, with this facilitating the operation of wrapping round the sub-valvular apparatus; at the same time, the anchoring member has a closed configuration when it is coupled to the framework, with this ensuring stability and sealing along the entire perimeter of the prosthetic member. In that case, it is further also possible to construct the anchoring member so as to be flexible in order to facilitate the connection thereof to the framework and/or to maintain the opening of the anchoring member so as to be larger in order to make its positioning easier in any situation, because this does not compromise the stability of the connection of the anchoring member with respect to the framework once it is closed.

In all the embodiments described above, the anchoring member may have a planar configuration, that is to say, with the centreline thereof extending in a plane, or a three-dimensional configuration, that is to say, with the centre line thereof extending in space. The flat configuration promotes structural simplicity and ease of connection of the prosthesis to the framework while the three-dimensional configuration promotes correspondence to the anatomy of the atrio-ventricular valve, for example, to the typical saddle-like shape of the annulus of the mitral valve.

As a further improvement, the anchoring member may be coated, at least in the portion thereof intended to come into contact with the valve leaflets, with a synthetic material, for example, fabric composed of PET or PTFE, or a polyurethane-containing or silicone-containing elastomer material, or with a biological material, for example, pericardium of animal origin; that material may cover the anchoring member in the form of films or may be extruded therewith. This reduces the tensions on the valve leaflets caused by the interference with the anchoring member and promotes integration of the anchoring member with the valve leaflets via a controlled endothelisation thereof.

Furthermore, the anchoring member and the framework may have different resilient properties.

For example, it is preferable for the anchoring member and the framework to be collapsible. In that manner, it is possible to reduce the overall dimensions thereof by compressing them during their introduction into the heart; they can then be expanded in order to regain their operating dimensions during a final step of the implantation procedure. That result may be achieved by manufacturing the various components of the prosthesis from a material which also has resilient behaviour in the configuration with a minimum spatial requirement thereof so that they are capable of automatically regaining the operating dimensions thereof by resilient return; in that case, the anchoring member and the framework can be constructed, for example, from an equiatomic alloy of nickel and titanium (known as Nitinol) or from other titanium alloys (such as Ti6Al4V). Alternatively or in combination, the same result may be achieved by constructing the structural components of the prosthesis from a material having plastic or elastoplastic characteristics, in such a manner that they can regain their operating dimensions owing to plastic deformation by means of an expansion device, such as an inflatable balloon; in that case, the anchoring member and the framework may be constructed, for example, from specific stainless steels (such as AISI 316LVM) or from alloys based on cobalt and chromium (such as L605). The above-described characteristic of the anchoring member and the framework allows the use of surgical access routes which have reduced dimensions, and which therefore are not very invasive, for example, using transcatheter percutaneous techniques. In any case, it is preferable for the anchoring member to be substantially non-extensible, that is to say that it cannot increase the length of the centre line thereof in order to ensure correct connection thereof to the framework. For example, under normal conditions of use, the length of the anchoring member (along the centre line) may increase, from when it is separate from the framework (that is to say, in a rest condition) to when it is coupled thereto (that is to say, in an operating condition), by a maximum of 5% and preferably a maximum of 3% (for example, from 1 to 2%).

However, the anchoring member and the framework may have different geometric characteristics (in terms of both dimensions and shape) in terms of the relationship which exists between the original geometric shapes (when separate) and final geometric shapes thereof (when coupled together).

In particular, the anchoring member is defined as being rigid if the final shape thereof is substantially equal to the original shape thereof, that is to say, if the shape thereof remains substantially unchanged when it is coupled to the framework. In that case, it is preferable for the framework to be capable of being completely adapted to the shape of the anchoring member over the entire extent thereof. However, the anchoring member is defined as being flexible if the final shape of the anchoring member is equal to the original shape of the framework, that is to say, it is completely adapted to the shape of the framework, also ensuring correct connection owing to the non-extensibility thereof. Finally, the anchoring member is defined as being semi-rigid if the final shape thereof is intermediate between the original shape of the anchoring member and the original shape of the framework, that is to say, if the final shapes of the anchoring member and the framework result from a balance between the resilient characteristics thereof. In that case, it is preferable for the framework and the anchoring member to be able to adapt over the entire extent thereof but without inhibiting the functionality of the prosthetic leaflets so as to assume a final common predetermined shape. For example, the flexible anchoring member may be constructed with wire rings (plain or interwoven), from fabric, with one or more inner portions composed of elastomer material, for example, based on silicone or polyurethane which is biologically compatible, or with a combination thereof. However, the semi-rigid or rigid anchoring member may be constructed from the same materials but reinforced with one or more inner portions of biologically compatible polymer material, for example, PET or PTFE, or metal, for example, Nitinol, or alloys based on titanium, cobalt or chromium.

The semi-rigid structural solution allows selection of an original shape for the anchoring member which makes it easier to position it, for example, a shape which is anatomically compatible with the cardiovascular valve and, at the same time, an original shape of the framework which simplifies the construction thereof, for example, circular. The final shape of the two components, once connected to each other, for example, oval, provides good anchoring of the prosthesis and good functionality of the prosthetic leaflets, for example, when the prosthetic member is provided with two prosthetic leaflets or three prosthetic leaflets with a sufficient height of coaptation.

In any case, the prosthetic member is subjected to controlled deformation which depends only marginally on the anatomical characteristics of the implantation site. This makes the implantation procedure reproducible and the functioning of the prosthesis predictable; in that manner, it is also possible to verify the correct functioning of the prosthesis in an effective and significant manner by means of laboratory tests or tests on animal models.

As a further improvement, the framework may have constructed separately, at least functionally, a support component for supporting the prosthetic leaflets and a connection component for connection to the anchoring member. That result can be achieved with physically different elements or with a single element having functionally different portions. The support component and the connection component are connected to each other by means of portions of an intermediate structure which are flexible and adaptable and which substantially reduce any geometric interaction between the support component and the connection component. For example, the intermediate structure may be formed by radial arms which are covered with one or more fabric films (of synthetic or biological material) which allow preservation of the sealing capacity along the entire perimeter of the prosthetic member.

In that manner, when the anchoring member is connected to the framework, the support component substantially always retains its original shape, independently of any deformations of the connection structure. Therefore, it is possible to independently optimize the shape of the support component, for example, circular, for good functionality of the valve leaflets, and the final shape of the connection component, for example, oval, when it is connected to a semi-rigid D-like anchoring member, for stable anchoring.

Furthermore, the framework and the anchoring member may have different dimensions in relation to the atrio-ventricular valve. For example, the framework and the anchoring member may be underdimensioned, that is to say, with a surface circumscribed by the centre line of the anchoring member having an area less than that of the atrio-ventricular orifice, for example, of from 70 to 90%. That structure provides an additional constraining effect on the atrio-ventricular valve that is advantageous in the event of prolapse of the annulus; furthermore, the invasiveness of the implantation procedure is reduced owing to the smaller dimensions of the prosthesis. Alternatively, the framework and the anchoring member may have the same dimensions as the atrio-ventricular valve with a surface circumscribed by the centre line of the anchoring member substantially equal to that of the atrio-ventricular orifice. That solution utilizes emodynamically the atrio-ventricular orifice fully; furthermore, it allows an improvement in the anchoring of the prosthesis with respect to the atrio-ventricular valve because it occurs in a portion of the valve leaflets near the annulus where it has maximum resistance and thickness.

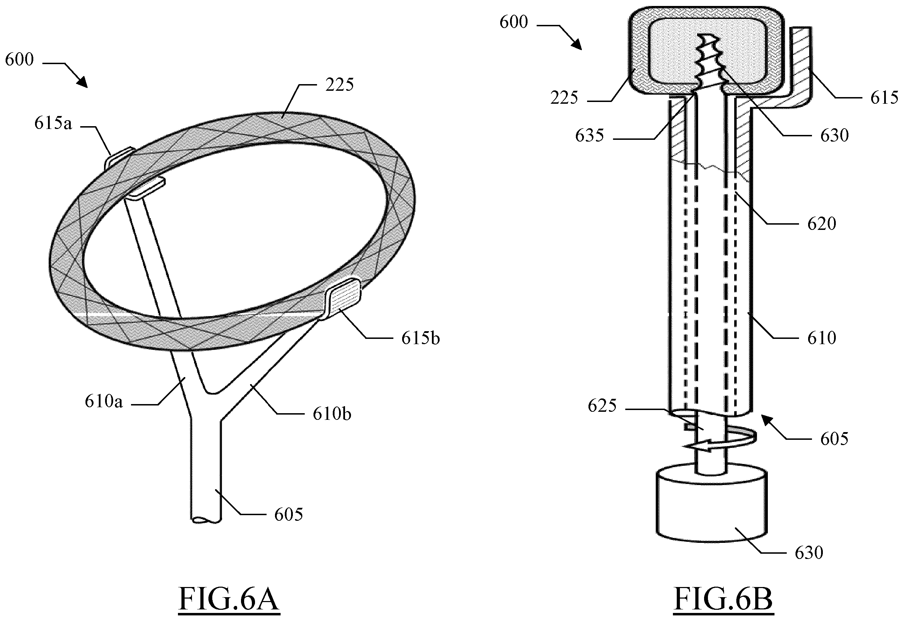

With reference to Figure 6A, there is shown a partial view of a delivery system 600 of the anchoring member.

The delivery system 600 comprises a shaft 605 which has a remote control and which is constructed from a material sufficiently flexible to allow insertion thereof in the heart and sufficiently rigid to allow control of its position in an effective manner. The shaft 605 is provided with a structure for supporting the ring 225 at the distal end thereof which comprises a fork formed by two arms 610a and 610b, each of which terminates in a cradle 615a and 615b, respectively; in particular, each cradle 615a, 615b comprises a support base (in order to support the ring 225) and an external containment wall (in order to prevent lateral movement of the ring 225). However, the cradle 615a, 615b does not have an internal containment wall so as to avoid any interference with the framework during the implantation procedure.

A detail of that delivery system 600 is shown in Figure 6B, in which each arm with the cradle thereof is generally designated 610 and 615, respectively. In that case, the shaft 605 is provided with a through-hole 620. An inner locking rod 625 is freely inserted in the through-hole 620. A proximal end of the inner locking rod 625 is provided with a control handle 630 which is used to rotate the inner locking rod 625 about a longitudinal axis thereof inside the through-hole 620 from the proximal end of the shaft 605; the inner locking rod 625 terminates at the distal end thereof in a threaded tip 630 which corresponds to a threaded hole 635 which is created in the ring 225, which threaded tip 630 projects from the arm 610 in the region of the cradle 615.

In that manner, when the threaded tip 630 of the inner locking rod 625 is screwed in the threaded hole 635 of the ring 225, the ring 225 is securely locked with respect to the delivery system 600; this allows the ring 225 to be supported in a stable manner during its positioning. Once the ring 225 has been coupled to the framework, the threaded tip 630 of the inner locking rod 625 is unscrewed from the threaded hole 635 of the ring 225; in that manner, the ring 225 is released, allowing the delivery system 600 to be removed. The same through-hole 620 can also be used (retrieving the inner locking rod 625 before the delivery system 600 is moved) in order to introduce the above-described closure wire (in the case of an anchoring structure with an open configuration).

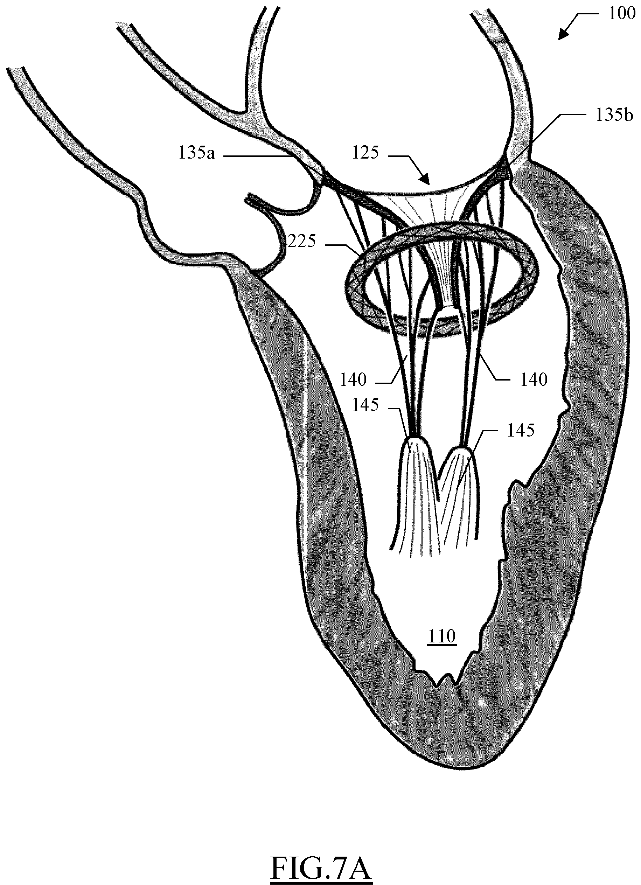

With reference to Figure 7A - Figure 7C, there are shown the main steps of a procedure for intra-ventricular implantation of the prosthesis in accordance with a form of the invention, in which the prosthesis is implanted in the left ventricle 110.

Starting from Figure 7A, the anchoring member (the ring 225 in the embodiment illustrated in the Figure) is positioned in the left ventricle 110 around the valve leaflets 135a, 135b; in that case, it is preferable for the ring 225 to have an open configuration so as to allow it to be positioned around the valve leaflets 135a, 135b even if they are connected to the internal wall of the left ventricle 110 by means of the chordae tendinae 140 and the papillary muscles 145.

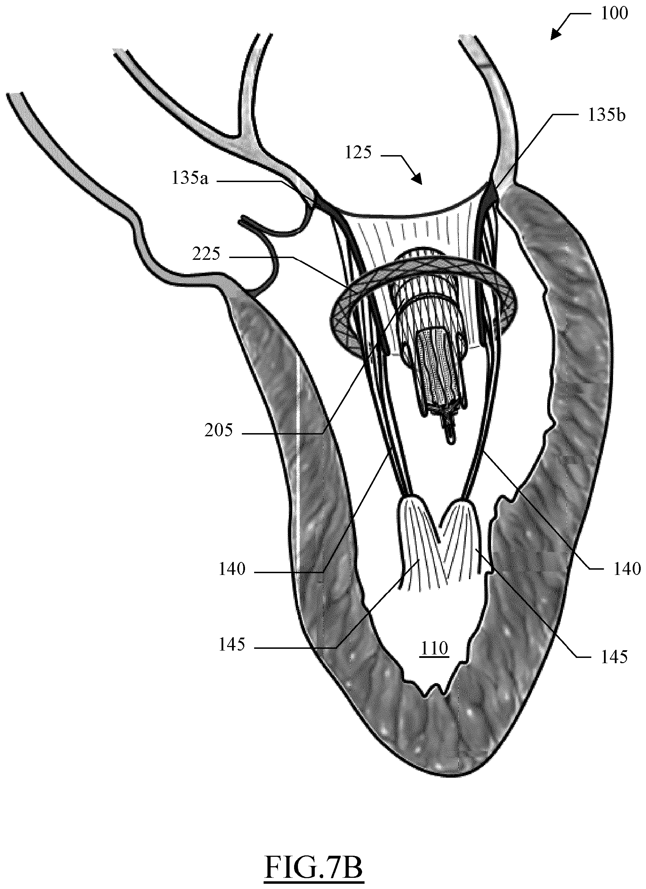

With reference to Figure 7B, the prosthetic member 205 is compressed and inserted inside the valve leaflets 135a, 135b at the height of the ring 225.

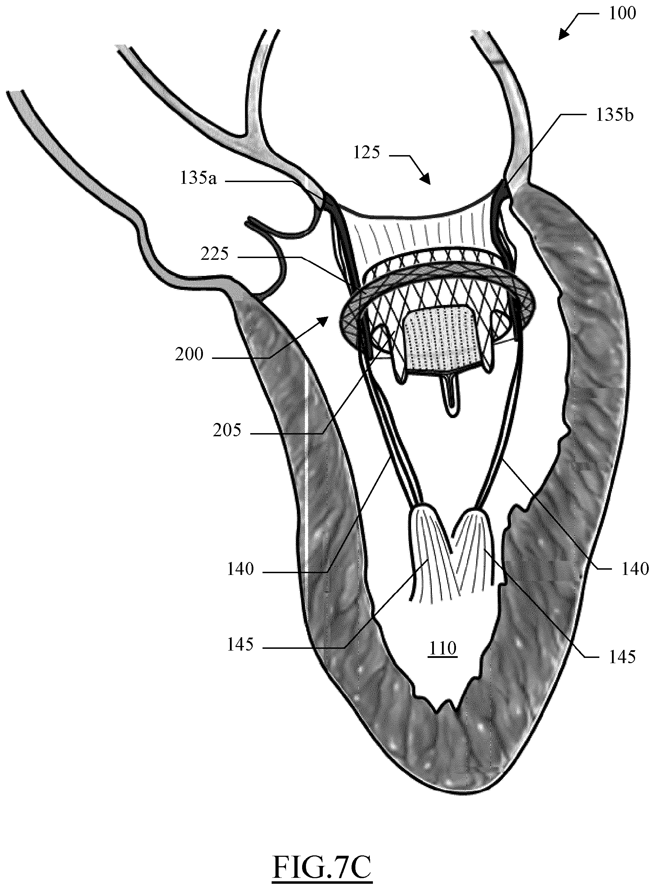

As shown in Figure 7C, the prosthetic member 205 expands (by means of resilient recovering or an expansion device) so as to regain its operating dimensions. Consequently, the ring 225 is coupled to the framework of the prosthetic member 205, locking and segregating a corresponding portion of the valve leaflets 135a, 135b between the framework of the prosthetic member 205 and the ring 225, thereby obtaining anchoring of the prosthesis 200 with respect to the mitral valve 125.

The intra-ventricular implantation procedure allows to preserve the integrity of the sub-valvular apparatus 140, 145 of the mitral valve 125, that is to say, the chordae tendinae 140 and the papillary muscles 145; this minimizes the invasiveness of the implantation procedure and the risks of dilative pathologies of the left ventricle 110.

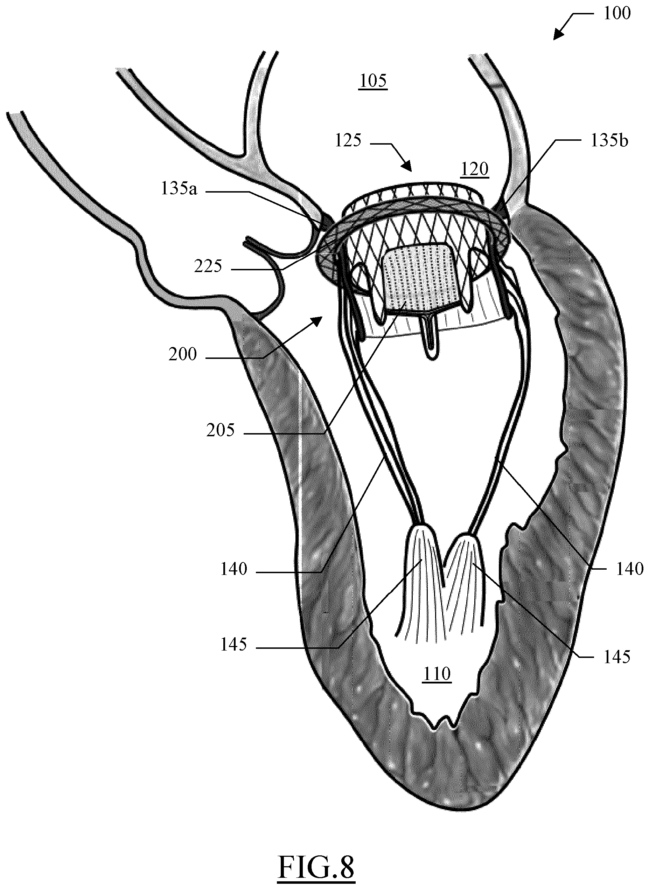

However, Figure 8 shows an embodiment of a sub-annular implantation of the prosthesis of the invention, in which it is implanted immediately below the annulus of the mitral valve 125 in the left ventricle 110 owing to anchoring with respect to the valve leaflets 135a, 135b.

The sub-annular implantation procedure is similar to the intra-ventricular procedure described above; in that case, however, the ring 225 is positioned immediately below the annulus of the mitral valve 125. Therefore, the portion of the valve leaflets 135a, 135b which remains locked between the framework of the prosthetic member 205 and the ring 225 is the portion which is immediately proximal to the insertion line on the annulus.

The sub-annular implantation procedure, in addition to preserve the integrity of the sub-valvular apparatus 140, 145 of the mitral valve 125 as above, improves the reliability of the anchoring of the prosthesis 200 with respect to the mitral valve 125, being clamping a portion of the valve leaflets 135a, 135b which normally has maximum resistance and thickness. In this manner, the prosthesis 200 is further provided in a physiological position so as to limit the projection thereof into the left ventricle 110. This also allows a reduction in the risk of interference by the valve leaflets 135a, 135b on the aortic systolic flow because the ring 225 behind the annulus constraints any movement of a portion thereof between it and the prosthesis 200.

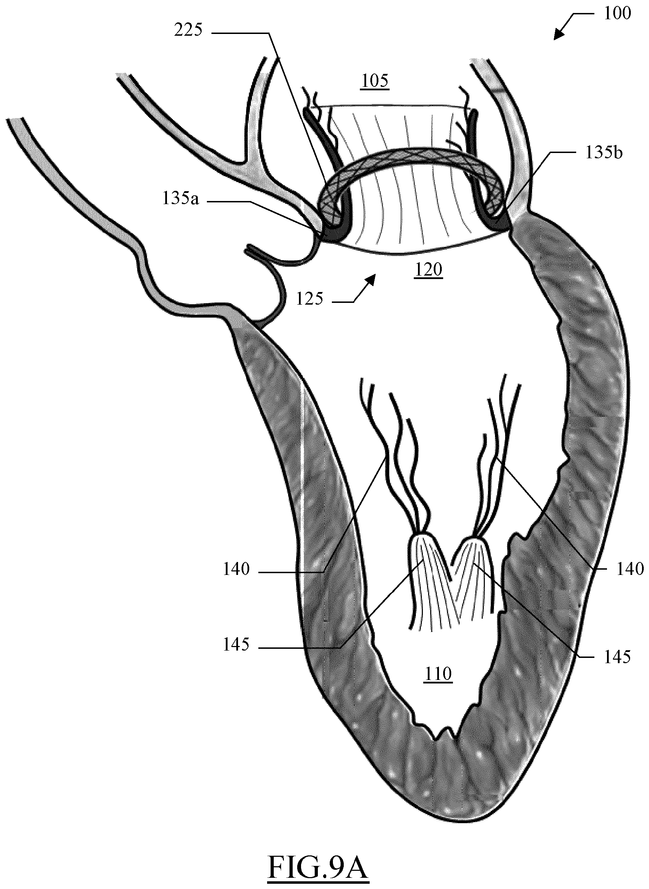

With reference to Figure 9A - Figure 9B, there are shown the main steps of a supra-annular implantation procedure of the prosthesis in which it is implanted immediately above the annulus of the mitral valve 125 in the left atrium 105.

Starting with Figure 9A, the anchoring member (the ring 225 in the embodiment illustrated in the Figure) is positioned in the left atrium 105 immediately above the annulus. The valve leaflets 135a, 135b are freed from the internal wall of the left ventricle 110, for example, by cutting the chordae tendinae 140; the valve leaflets are then turned over inside the left atrium 105 so as to extend over the ring 225 which completely surrounds them. That result may be achieved at the same time as the compressed valve member (not shown in the Figure) is positioned inside the ring 225. In particular, the valve member is inserted in the left ventricle 110 and is then moved to the left atrium 105 via the left atrio-ventricular orifice 120; the same system for delivery the valve member also drags the valve leaflets 135a, 135b inside the left atrium 105 so that they are arranged between the valve member and the ring 225.

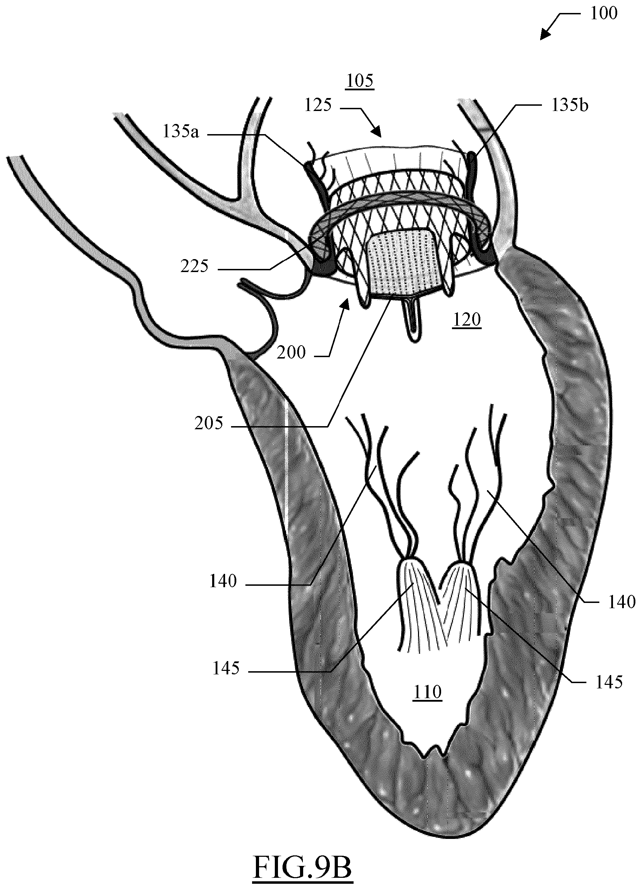

In Figure 9B, the prosthetic member 205 expands (by means of resilient return or an expansion device) so as to regain its operating dimensions. Consequently, the ring 225 is coupled to the framework of the prosthetic member 205 and a corresponding portion of the valve leaflets 135a, 135b is locked between the framework of the prosthetic member 205 and the ring 225 - thereby obtaining anchoring of the prosthesis 200 with respect to the mitral valve 125.

The supra-annular implantation procedure may also be implemented with a closed configuration of the ring 225. Furthermore, the removal of the valve leaflets 135a, 135b from the left ventricle 110 completely eliminates any risk of interference with the aortic systolic flow. The positioning of the ring 225 in the left atrium 105 is also advantageous owing to the anatomical characteristics thereof (for example, absence of the sub-valvular apparatus); furthermore, in the case of a transcatheter procedure, the access to the left atrium 105 is easier, for example, via trans-septal access from the right atrium, readily achievable via one of the venae cavae.

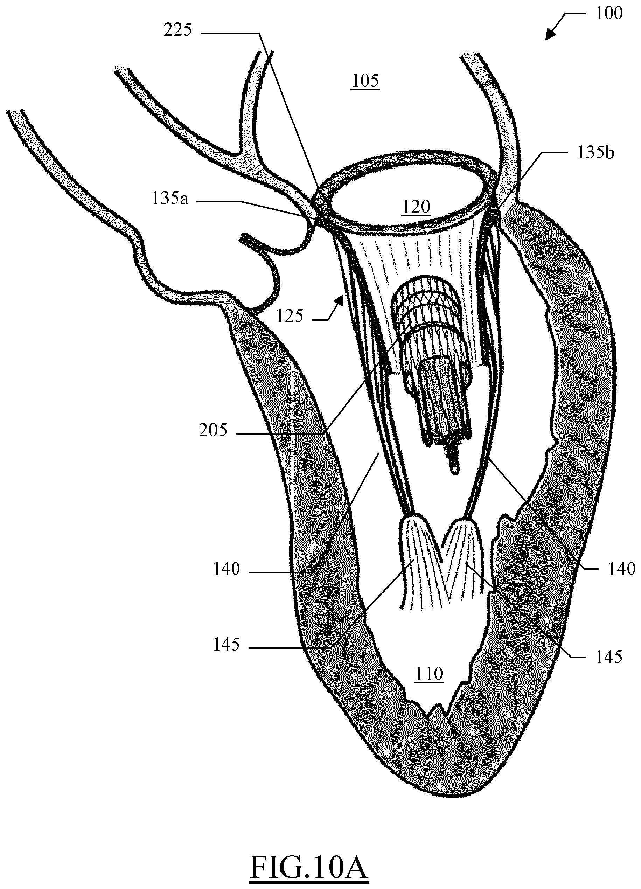

Figure 10A - Figure 10C show the main steps of another supra-annular implantation method for the prosthesis in accordance with an embodiment of the invention. The supra-annular implantation procedure described here differs from the procedure described in Figure 9A - Figure 9B because this variant allows the integrity of the sub-valvular apparatus of the mitral valve to be preserved without requiring the cutting of the chordae tendinae 140.

Starting from Figure 10A, the anchoring member 225 is positioned in the left atrium 105 immediately above the annulus. The prosthetic member 205 is compressed and inserted inside the valve leaflets 135a, 135b in a low position with respect to the plane of the annulus of the mitral valve 125 in the Figure.

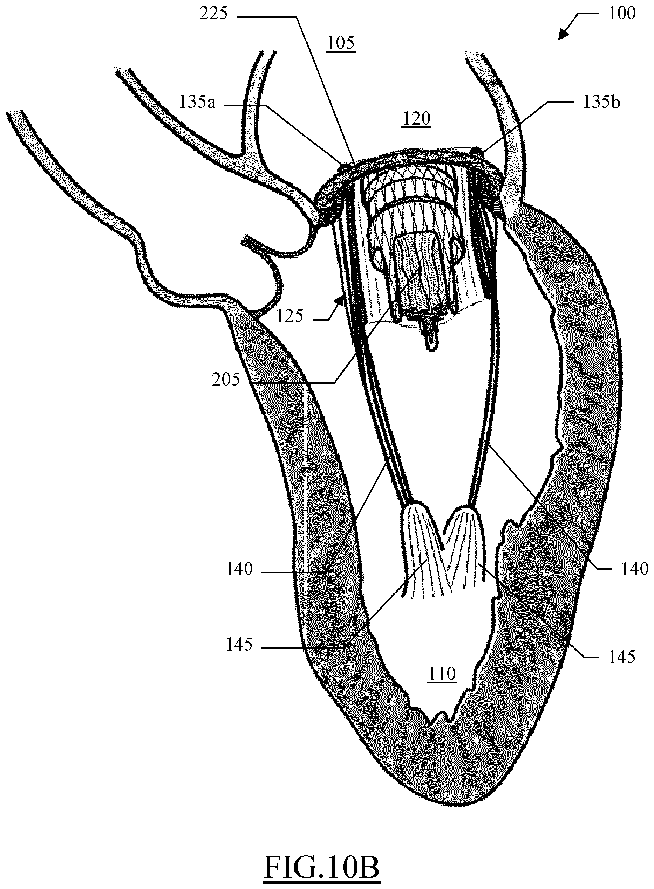

With reference to Figure 10B, the prosthetic member 205 is positioned with the outer groove at the height of the ring 225. At the same time, the valve leaflets 135a, 135b, also remaining secured to the papillary muscles 145 via the chordae tendinae 140 which are kept intact, are folded in the region of the insertion zone on the annulus, inside the ring 225. That arrangement of the valve leaflets 135a, 135b may be obtained using various techniques. A first technique provides for the use of specific hook-shaped surgical instruments which are known per se and which are already used to inspect the valve leaflets 135a, 135b, especially during procedures for repairing the mitral valve 125. Alternatively, it is possible to provide that the same system for delivering the prosthetic member 205 is capable to drag the valve leaflets 135a, 135b towards the left atrium 105. A third technique provides that the prosthetic member 205 is initially positioned inside the left ventricle 110, that is to say, in a lower position with respect to the implantation position. Therefore, the prosthetic member 205 is expanded, at least partially, until it engages with the valve leaflets 135a, 135b. Movement towards the left atrium 105 of the valve member 205 and positioning thereof in the region of the ring 225 bring about dragging of the valve leaflets 135a, 135b towards the left atrium 105, with resultant folding thereof inside the ring 225. In order to make that drag action more efficient, the outer lateral surface of the prosthetic member 205 may be provided with an engagement structure, for example, having teeth or hooks, able to become mechanically engaged with the valve leaflets 135a, 135b.

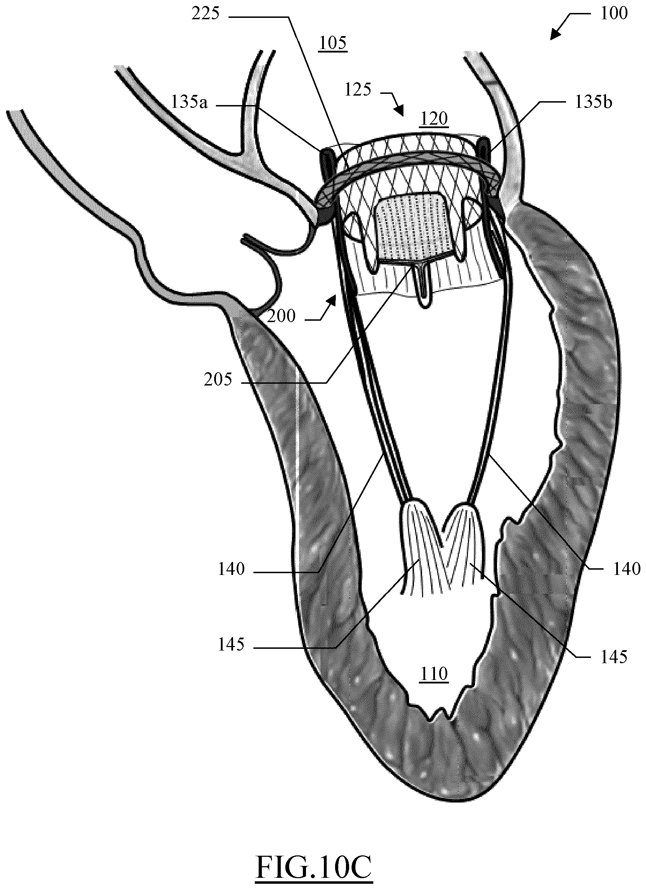

As shown in Figure 10C, the prosthetic member 205 completes its expansion so as to regain its operating dimensions. Consequently, the anchoring member 225 is coupled to the framework of the prosthetic member 205, that is to say, it is seated in the groove thereof in the embodiment in question; in that manner, the portion of the valve leaflets 135a, 135b folded inside the anchoring member 225 remains locked between the framework of the prosthetic member 205 and the ring 225, bringing about the anchoring of the prosthesis 200 with respect to the mitral valve 125.

This supra-annular implantation procedure retains the above-described advantages for Figure 9A - Figure 9B, but with the entire sub-valvular apparatus 140, 145 of the mitral valve 125 being kept intact.

Naturally, in order to satisfy contingent and specific requirements, a person skilled in the art may apply to the above-described solution a number of logical and/or physical modifications and variants.

The above-described prosthesis is also suitable for being used for the other heart valves, for example, the tricuspid valve, and more generally for any other human or animal cardiovascular valve (for example, for valves of peripheral haematic veins).

The above-described examples of shape, configuration, resilience and dimensions of the support structure and the anchoring member are merely illustrative and must not be intended to be exhaustive.

The prosthesis may be based on any other valve structure, even one without prosthetic leaflets, for example, with a ball, disc or half-discs.

Furthermore, in the case of an open anchoring member, it may also remain open after it has been connected to the support structure if it is sufficiently rigid and/or the opening thereof is sufficiently small.

The original and final shapes of the support member and the anchoring structure described above are merely by way of example; it is further possible for the final shapes both of the framework and of the anchoring member to be equal to the corresponding original shapes or only the final shape of the framework, or only the final shape of the anchoring member, or both to be different from the corresponding original shapes.

## Claims

1. A prosthesis for a cardiac atrio-ventricular valve, comprising valve leaflets, the prosthesis comprising:
- a prosthetic member (205) comprising a support structure (210) having a peripheral wall which defines a through-hole,
- a valve structure which is supported by the support structure (210) and which comprises prosthetic leaflets (220a-220c) which are configured to selectively close the through-hole of the support structure (210) so as to restore, during use, the functionality of a unidirectional valve,
and
- an anchoring member (225)
wherein the anchoring member (225) and the support structure (210) of the prosthetic member (205) are configured so as to be coupled each other, after implantation in the body of a patient, co-operating in order to entrap and lock the valve leaflets (135a - 135b) of the cardiac atrio-ventricular valve of the patient between the peripheral wall of the support structure (210) and the anchoring member (225) so as to keep the prosthesis (200) in position,
wherein a seating (230) is defined on the support structure (210), conjugate to the anchoring member (225), the support structure (210) and the anchoring member (225) being configured and having dimensions so that they are connected with interference so as to lock the anchoring member (225) in the seating (230), and wherein
the prosthetic member (205) and the anchoring member (225) being configured to be arranged at opposite sides of valve leaflets so that when the anchoring member (225) is matched to the support structure (210), said valve leaflets remain locked between the support structure (210) and the anchoring member (225).

2. A prosthesis according to claim 1, wherein the support structure (210) comprises at least one face (427B; 427C) which is folded about itself in order to form at least one lip (428B, 429B; 428C) which defines a corresponding edge of the seating (430B; 430C).

3. A prosthesis according to claim 1 or claim 2, wherein the support structure (210) is provided with protrusions (426A) which project radially therefrom to define at least one edge of the seating (430A; 430C).

4. A prosthesis according to claim 3, wherein each protrusion (426A) has a C-like shape which is defined by two opposing fingers, the protrusions being successively arranged along a peripheral wall of the support structure (210) so as to define the outline of a channel (430A) which brings about the seating of the anchoring member (225).

5. A prosthesis according to any one of the preceding claims, wherein the prosthetic member (205) comprises an engagement structure which leans out externally from the peripheral wall and which is capable of engaging, during the implantation of the prosthesis in the body of the patient, with the valve leaflets (135a - 135b) of the cardiac atrio-ventricular valve in order to drag them inside the anchoring member (225) where they are locked between the anchoring member (225) and the peripheral wall of the support structure (210).

6. A prosthesis according to any one of the preceding claims, wherein the seating (230) is a circumferential groove (226) which is formed on an outer surface of the peripheral wall of the support structure (210).

7. A prosthesis according to any one of the preceding claims, wherein the anchoring member (210) is connected to the support structure (210) in a stable manner.

8. A prosthesis according to any one of preceding claims, wherein the the anchoring member (225) is a ring, preferably a substantially circular ring, and/or the anchoring member (225) is collapsible.

9. A prosthesis according to any one of the preceding claims, wherein the anchoring member (225) comprises a main structure (530, 545, 550) having free ends which can be closed to bring the anchoring member from an open configuration to a closed configuration having a structural continuity.

10. A prosthesis according to claim 9, wherein the main structure (530, 545, 550) of the anchoring member may be closed by a closure structure to restore the structural continuity to the anchoring member (525F), and wherein preferably the main structure (530) of the anchoring member is tubular and comprises a through-hole which extends over the entire extent thereof, the closure structure (535-537) comprising a filiform structure (535).

11. A prosthesis according to claim 9 or claim 10, wherein the main structure (530) of the anchoring member may be closed by means of a closure wire (535), preferably by tensioning the closure wire (535).

12. A prosthesis according to any one of the preceding claims, wherein the support structure (210) and the anchoring member (225) have original shapes when they are separated from each other and final shapes, which are different from the original ones, when they match each other, respectively.

13. A prosthesis according to any one of the preceding claims, wherein the support structure (210) and the anchoring member (225) are collapsible in order to facilitate introduction inside the body of a patient and are expandable owing to the effect of resilient return, plastically or in a resilient/plastic manner, during use, in order to lock the valve leaflets (135a - 135b) of the cardiac atrio-ventricular valve between the expanded anchoring member (225) and the expanded support structure (210).

14. A prosthesis according to any one of the preceding claims, wherein the anchoring member (225) may be coated, at least in the portion thereof intended to come into contact with the valve leaflets (135a - 135b), with a synthetic material or with a biological material, and/or wherein the anchoring member (225) is reinforced with one or more inner portions of biologically compatible polymer material, or metal, or alloys based on titanium, cobalt or chromium.

15. A kit for restoring the functionality of a cardiac atrio-ventricular valve comprising a prosthesis (200) according to any one of claims 1 to 14 and instructions for introducing, positioning and implanting the prosthesis (200) in the body of a patient in such a manner as to entrap and lock the valve leaflets (135a - 135b) of the cardiac atrio-ventricular valve of the patient between the peripheral wall of the support structure (210) and the anchoring member (225) so as to keep the prosthesis (200) in position.

## Patentansprüche

1. Prothese für eine atrioventrikuläre Herzklappe mit Herzklappenblättchen, wobei die Prothese umfasst:
ein prothetisches Element (205) umfassend eine Stützstruktur (210) mit einer Umfangswand, die eine Durchgangsöffnung definiert,
eine Klappenstruktur, die durch die Stützstruktur (210) gestützt wird und prothetische Blättchen (220a - 220c) aufweist, die ausgebildet sind zum selektiven Schließen der Durchgangsöffnung der Stützstruktur (210), um bei Gebrauch die Funktionalität einer Einwegklappe wiederherzustellen, und ein Verankerungselement (225),
wobei das Verankerungselement (225) und die Stützstruktur (210) des prothetischen Elements (205) dazu ausgebildet sind, nach der Implantation in den Körper eines Patienten miteinander verbunden zu werden, und zusammenwirken um die Klappenblättchen (135a - 135b) der atrioventrikulären Herzklappe des Patienten zwischen der Umfangswand der Stützstruktur (210) und dem Verankerungselement (225) einzuschließen und zu arretieren, um die Prothese (200) in Position zu halten,
wobei an der Stützstruktur (210) eine Aufnahme (230) definiert ist, die zum Verankerungselement (225) konjugiert ist, wobei die Stützstruktur (210) und das Verankerungselement (225) derart ausgebildet sind und derartige Abmessungen aufweisen, dass sie durch Presspassung miteinander verbunden sind, um das Verankerungselement (225) in der Aufnahme (230) zu arretieren, und wobei
das prothetische Element (205) und das Verankerungselement (225) dazu ausgebildet sind, an gegenüberliegenden Seiten von Klappenblättchen angeordnet zu werden, sodass, wenn das Verankerungselement (225) an die Stützstruktur (210) angepasst wird, die Klappenblättchen zwischen der Stützstruktur (210) und dem Verankerungselement (225) arretiert bleiben.

2. Prothese nach Anspruch 1, wobei die Stützstruktur (210) wenigstens eine Stirnseite (427B; 427C) umfasst, die auf sich selbst gefaltet ist, um wenigstens eine Lippe (428B, 429B; 428C) zu bilden, die einen entsprechende Rand der Aufnahme (430B; 430C) definiert.

3. Prothese nach Anspruch 1 oder Anspruch 2, wobei die Stützstruktur (210) mit Vorsprüngen (426A) versehen ist, die radial von dieser vorstehen, um wenigstens einen Rand der Aufnahme (430A; 430C) zu definieren.

4. Prothese nach Anspruch 3, wobei jeder Vorsprung (426A) eine C-förmige Form aufweist, die durch zwei gegenüberliegende Finger definiert ist, wobei die Vorsprünge (426A) nacheinander entlang einer Umfangswand der Stützstruktur (210) angeordnet sind, um den Umriss eines Kanals (430A) zu definieren, der die Aufnahme des Verankerungselements (225) bildet.

5. Prothese nach einem der vorhergehenden Ansprüche, wobei das prothetische Element (205) ferner eine Eingriffsstruktur hat, die sich von der Umfangswand zur Außenseite hinauslehnt und die geeignet ist für den Eingriff mit den Klappenblättchen (135a - 135b) der atrioventrikulären Herzklappe während der Implantation der Prothese in den Körper eines Patienten, um die Klappenblättchen (135a - 135b) in das Verankerungselement (225) zu ziehen, wo sie zwischen dem Verankerungselement (225) und der Umfangswand der Stützstruktur (210) arretiert werden.

6. Prothese nach einem der vorhergehenden Ansprüche, wobei die Aufnahme (230) eine umlaufende Nut (226) ist, die an einer Außenfläche der Umfangswand der Stützstruktur (210) ausgebildet ist.

7. Prothese nach einem der vorhergehenden Ansprüche, wobei das Verankerungselement (225) auf stabile Weise mit der Stützstruktur (210) verbunden ist.

8. Prothese nach einem der vorhergehenden Ansprüche, wobei das Verankerungselement (225) ein Ring, vorzugsweise ein im Wesentlichen kreisförmiger Ring, ist und/oder wobei das Verankerungselement (225) zusammenklappbar ist.

9. Prothese nach einem der vorhergehenden Ansprüche, wobei das Verankerungselement (225) eine Hauptstruktur (530, 545, 550) mit freien Enden umfasst, die geschlossen werden können, um das Verankerungselement (225) aus einer offenen Konfiguration in eine geschlossene Konfiguration mit struktureller Kontinuität zu bringen.

10. Prothese nach Anspruch 9, wobei die Hauptstruktur (530, 545, 550) des Verankerungselements (225) durch eine Verschlussstruktur (535-537) verschlossen werden kann, um die strukturelle Kontinuität des Verankerungselements (525F) wiederherzustellen, wobei vorzugsweise die Hauptstruktur (530) des Verankerungselements (225) rohrförmig ist und eine Durchgangsöffnung aufweist, die sich über ihre gesamte Ausdehnung erstreckt, und wobei die Verschlussstruktur (535-537) eine fadenförmige Struktur (535) umfasst.

11. Prothese nach Anspruch 9 oder Anspruch 10, wobei die Hauptstruktur (530) des Verankerungselements (225) mittels eines Verschlussdrahtes (535) geschlossen werden kann, vorzugsweise durch Spannen des Verschlussdrahtes (535).

12. Prothese nach einem der vorhergehenden Ansprüche, wobei die Stützstruktur (210) und das Verankerungselement (225) ursprüngliche Formen aufweisen, wenn sie voneinander getrennt sind, und endgültige Formen, die sich von den ursprünglichen unterscheiden, wenn sie aufeinander abgestimmt sind.

13. Prothese nach einem der vorhergehenden Ansprüche, wobei die Stützstruktur (210) und das Verankerungselement (225) zusammendrückbar sind, um deren Einführen in den Körper eines Patienten zu erleichtern, und sich aufgrund der elastischen Rückstellwirkung plastisch oder elastisch/plastisch ausdehnen können, um die Klappenblättchen (135a - 135b) der atrioventrikulären Herzklappe zwischen dem ausgedehnten Verankerungselement (225) und der ausgedehnten Stützstruktur (210) zu arretieren.

14. Prothese nach einem der vorhergehenden Ansprüche, wobei das Verankerungselement (225) zumindest in dem Abschnitt, der mit den Klappenblättchen (135a - 135b) in Kontakt kommen soll mit einem synthetischen Material oder einem biologischen Material beschichtet sein kann, und/oder wobei das Verankerungselement (225) mit einem oder mehreren inneren Abschnitten aus biologisch verträglichem Polymermaterial oder Metall oder Legierungen auf Titan-, Kobalt- oder Chrombasis verstärkt ist.

15. Kit zum Wiederherstellen der Funktionalität einer atrioventrikulären Herzklappe, umfassend eine Prothese (200) nach einem der Ansprüche 1 bis 14 und Anweisungen zum Einführen, Positionieren und Implantieren der Prothese (200) in den Körper eines Patienten derart, dass die Klappenblättchen (135a - 135b) der atrioventrikulären Herzklappe des Patienten zwischen der Umfangswand des Stützstruktur (210) und dem Verankerungselement (225) eingeschlossen und arretiert werden, um die Prothese (200) in ihrer Position zu halten.

## Revendications

1. Prothèse pour valve auriculo-ventriculaire cardiaque, comprenant des feuillets de valve, la prothèse comprenant :
- un organe prothétique (205) comprenant une structure de support (210) ayant une paroi périphérique qui définit un trou traversant,
- une structure de valve qui est supportée par la structure de support (210) et qui comprend des feuillets prothétiques (220a-220c) qui sont configurés pour fermer sélectivement le trou traversant de la structure de support (210) de manière à restaurer en cours d'utilisation la fonctionnalité d'une valve unidirectionnelle,
et
- un organe d'ancrage (225)
l'organe d'ancrage (225) et la structure de support (210) de l'organe prothétique (205) étant configurés pour s'accoupler mutuellement, après implantation dans le corps d'un patient, coopérant pour emprisonner et verrouiller les feuillets de valve (135a - 135b) de la valve auriculo-ventriculaire cardiaque du patient entre la paroi périphérique de la structure de support (210) et l'organe d'ancrage (225) pour maintenir la prothèse (200) en position,
un siège (230) étant défini sur la structure de support (210), conjugué à l'organe d'ancrage (225), la structure de support (210) et l'organe d'ancrage (225) étant configurés et présentant des dimensions de sorte qu'ils sont reliés par interférence afin de bloquer l'organe d'ancrage (225) dans le siège (230), et
l'organe prothétique (205) et l'organe d'ancrage (225) étant configurés pour être agencés sur des côtés opposés des feuillets de valve, de sorte que lorsque l'organe d'ancrage (225) est adapté à la structure de support (210), lesdits feuillets de valve restent bloqués entre la structure de support (210) et l'organe d'ancrage (225).

2. Prothèse selon la revendication 1, la structure de support (210) comprenant au moins une face (427B ; 427C) qui est repliée sur elle-même afin de former au moins une lèvre (428B ; 429B ; 428C) qui définit un bord correspondant du siège (430B ; 430C).

3. Prothèse selon la revendication 1 ou la revendication 2, la structure de support (210) étant munie de saillies (426A) qui font saillie radialement à partir de celle-ci pour définir au moins un bord du siège (430A ; 430C).

4. Prothèse selon la revendication 3, chaque saillie (426A) ayant une forme en C définie par deux doigts opposés, les saillies étant agencées successivement le long d'une paroi périphérique de la structure de support (210) de manière à définir le contour d'un canal (430A) provoquant l'appui de l'organe d'ancrage (225).

5. Prothèse selon l'une quelconque des revendications précédentes, l'organe prothétique (205) comprenant une structure de mise en prise qui s'appuie extérieurement à partir de la paroi périphérique et qui est apte à venir en prise, pendant l'implantation de la prothèse dans le corps du patient, avec les feuillets de valve (135a - 135b) de la valve auriculo-ventriculaire cardiaque afin de les entraîner à l'intérieur de l'organe d'ancrage (225) où ils sont bloqués entre l'organe d'ancrage (225) et la paroi périphérique de la structure de support (210).

6. Prothèse selon l'une quelconque des revendications précédentes, le siège (230) étant une rainure circonférentielle (226) formée sur une surface extérieure de la paroi périphérique de la structure de support (210).

7. Prothèse selon l'une quelconque des revendications précédentes, l'organe d'ancrage (210) étant relié de manière stable à la structure de support (210).

8. Prothèse selon l'une quelconque des revendications précédentes, l'organe d'ancrage (225) étant un anneau, de préférence un anneau sensiblement circulaire, et/ou l'organe d'ancrage (225) étant repliable.

9. Prothèse selon l'une quelconque des revendications précédentes, l'organe d'ancrage (225) comprenant une structure principale (530, 545, 550) ayant des extrémités libres qui peuvent être fermées pour amener l'organe d'ancrage d'une configuration ouverte à une configuration fermée ayant une continuité structurelle.

10. Prothèse selon la revendication 9, la structure principale (530, 545, 550) de l'organe d'ancrage pouvant être fermée par une structure de fermeture pour restaurer la continuité structurelle de l'organe d'ancrage (525F), la structure principale (530) de l'organe d'ancrage étant de préférence tubulaire et comprenant un trou traversant qui s'étend sur toute son étendue, la structure de fermeture (535-537) comprenant une structure filiforme (535).

11. Prothèse selon la revendication 9 ou la revendication 10, la structure principale (530) de l'organe d'ancrage pouvant être fermée au moyen d'un fil de fermeture (535), de préférence en tendant le fil de fermeture (535).

12. Prothèse selon l'une quelconque des revendications précédentes, la structure de support (210) et l'organe d'ancrage (225) ayant des formes originales lorsqu'ils sont séparés l'un de l'autre et des formes finales, qui sont respectivement différentes des formes originales, lorsqu'ils correspondent l'un à l'autre.

13. Prothèse selon l'une quelconque des revendications précédentes, la structure de support (210) et l'organe d'ancrage (225) étant repliables pour faciliter l'introduction à l'intérieur du corps d'un patient et qui sont expansibles sous l'effet d'un retour élastique, plastiquement ou de manière élastique/plastique, pendant l'utilisation, afin de verrouiller les feuillets de valve (135a - 135b) de la valve auriculo-ventriculaire cardiaque entre l'organe d'ancrage expansé (225) et la structure de support expansée (210).

14. Prothèse selon l'une quelconque des revendications précédentes, l'organe d'ancrage (225) pouvant être revêtu, au moins dans sa partie destinée à venir en contact avec les feuillets de valve (135a - 135b), d'un matériau synthétique ou d'un matériau biologique, et/ou l'organe d'ancrage (225) étant renforcé par une ou plusieurs parties intérieures de matériau polymère biologiquement compatible, ou de métal ou d'alliages à base de titane, de cobalt ou de chrome.

15. Kit pour restaurer la fonctionnalité d'une valve auriculo-ventriculaire cardiaque comprenant une prothèse (200) selon l'une quelconque des revendications 1 à 14 et des instructions pour introduire, positionner et implanter la prothèse (200) dans le corps d'un patient de manière à emprisonner et verrouiller les feuillets de valve (135a - 135b) de la valve auriculo-ventriculaire cardiaque du patient entre la paroi périphérique de la structure de support (210) et l'organe d'ancrage (225) de manière à maintenir la prothèse (200) en position.
